Europäisches Patentamt

European Patent Office

Office européen des brevets

(11) Publication number: **0 277 505 B1**

# EUROPEAN PATENT SPECIFICATION

(45) Date of publication of patent specification: **22.04.92**  (51) Int. Cl.<sup>5</sup>: **A61L 2/10**

(21) Application number: **88100402.2**

(22) Date of filing: **13.01.88**

(54) **Apparatus for sterilization with ultraviolet light.**

(30) Priority: **14.01.87 JP 7299/87**

(43) Date of publication of application:
**10.08.88 Bulletin 88/32**

(45) Publication of the grant of the patent:
**22.04.92 Bulletin 92/17**

(84) Designated Contracting States:
**DE FR GB SE**

(56) References cited:
**EP-A- 0 143 856**
**GB-A- 1 093 751**
**GB-A- 2 155 625**

(73) Proprietor: **SUMITOMO ELECTRIC INDUSTRIES LIMITED**
**No. 15, Kitahama 5-chome, Higashi-ku**
**Osaka-shi, Osaka 541(JP)**

(72) Inventor: **Mogi, Masaharu c/o Yokohama**
**Works of Sumitomo**
**Electric Ind. Ltd. No. 1, Taya-cho Sakae-ku**
**Yokohama-shi Kanagawa(JP)**

(74) Representative: **Patentanwälte Grünecker,**
**Kinkeldey, Stockmair & Partner**
**Maximilianstrasse 58**
**W-8000 München 22(DE)**

## Description

The present invention relates to a sterilization apparatus which utilizes ultraviolet light and which is particularly intended to be employed for medical and industrial purposes.

## BACKGROUND OF THE INVENTION

The sterilization action of ultraviolet light has been known for many years. Since sterilizing with ultraviolet light is quicker than chemical or thermal sterilization and since it does not pollute the working environment either during or after sterilization, sterilization with ultraviolet light is extensively used not only in the medical field but also in industrial fields. Ultraviolet light commonly used for sterilization is in the ultaviolet region comprising wavelengths between 200 and 300 nm because DNA and proteins in bacterial cells have absorption peaks in the neighborhood of 260 nm. Low-pressure mercury-vapor lamps, which have a spectrum line at 254 nm, have been extensively used as sterilizing lamps.

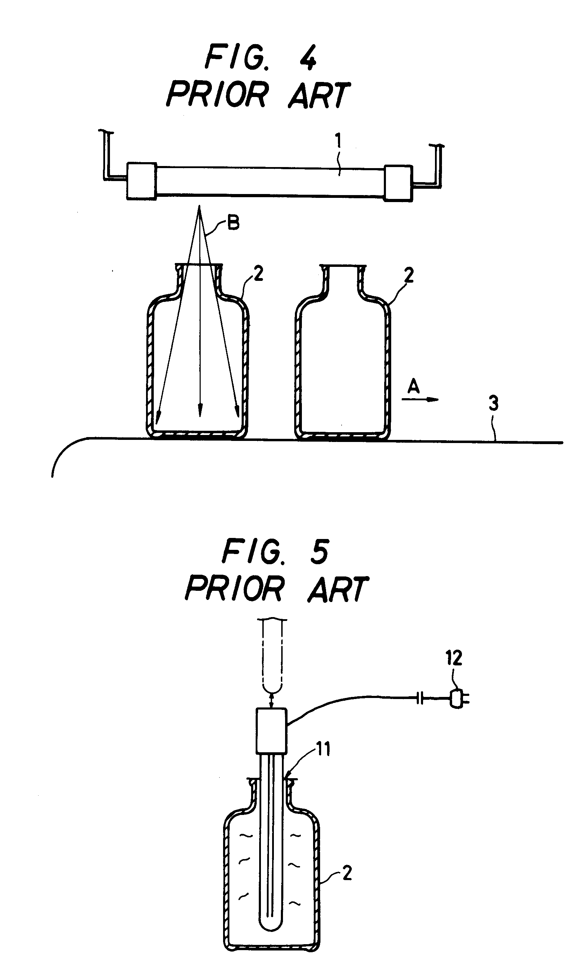

Fig. 4 depicts a conventional method of sterilization with ultraviolet light. As shown, a low-pressure mercury-vapor lamp 1 is placed horizontally above a plurality of bottles 2 which will travel on a line 3 in the direction indicated by arrow A. In this arrangement, the ultraviolet light from the low-pressure mercury-vapor lamp 1 is directed into the bottles 2 as indicated by arrow B so as to sterilize the interior of the bottles by ultraviolet light.

However, the amount of ultraviolet light that is introduced into the bottles 2 by this method is small and therefore the efficiency of this sterilization process is low. A particular problem associated with this method is that many portions of the interior of a bottle 2 are left unirradiated with ultraviolet light if the opening of the bottle is small or if the inner surface is not flat but has high and low spots.

In order to solve these problems, it has been proposed that the output power of the low-pressure mercury-vapor lamp be increased. However, this increases the costs of the sterilization process. Furthermore, some parts of the interior of the bottles 2 still remain unirradiated with ultraviolet light.

To overcome these disadvantages, it has been proposed that a low-pressure mercury-vapor lamp which is designed to have a special shape, for example, a pen shape, be inserted through the opening of a bottle 2 which is to be sterilized. This method is shown schematically in Fig. 5. As shown, a slender pen-shaped low-pressure mercury-vapor lamp 11 is supplied with electric power through a plug 12 and is inserted into the bottle 2 through its opening. In this way, the ultraviolet light from the lamp 11 is efficiently applied to the entire inner surface of the bottle 2.

However, the method shown in Fig. 5 still has some problems. First of all, the pen-shaped low-pressure mercury-vapor lamp has a certain diameter and is difficult to insert into a bottle 2 having a small opening in the top. Secondly, the low-pressure mercury-vapor lamp might touch the bottle or some other object and break causing an accident such as contamination by mercury vapor.

## SUMMARY OF THE INVENTION

An object of the present invention is to provide an apparatus that is capable of sterilizing bottles and other objects with ultraviolet light in a reliable and safe manner.

These and other objects are achieved by an apparatus for sterilization with ultraviolet light according to the present invention comprising a light guide having a receiving end surface and an exit end surface, an ultraviolet lamp and a reflector mirror which reflects a predetermined wavelength of ultraviolet radiation from the ultraviolet lamp and concentrates the reflected radiation at the receiving end surface of the light guide. The light emerging from the exit end surface of the light guide is directed on an object to be sterilized. The light guide may have a plurality of optical fibers.

In the sterilization apparatus of the present invention having the construction as described above, the reflector mirror concentrates ultraviolet light on the receiving end surface of the light guide. The light guide directs the incident ultraviolet light to travel to its exit end surface emerging therefrom to be directed to an object to be sterilized. Because of this feature of concentrating the ultraviolet light, the apparatus of the present invention is capable of sterilizing bottles, and other objects with ultraviolet light in a reliable and safe manner.

If a bundle of optical fibers is used as a light guide, the object to be sterilized can be irradiated with ultraviolet light in a precise manner and in an environment that needs no special precaution against hazard such as explosion. The apparatus of the present invention is useful not only for medical applications but also for other applications such as food, cosmetic and pharmaceutical applications.

## BRIEF DESCRIPTION OF THE DRAWINGS

FIGURE 1 is a schematic diagram of an apparatus for sterilization with ultraviolet light according to one embodiment of the present invention;

FIGURE 2 shows schematically two experimental systems employed for verifying the effectiveness of the apparatus of the present invention;

FIGURE 3 is a graph showing the results of experiments conducted with the systems shown in FIGURE 2; and

FIGURES 4 and 5 show schematically two conventional art apparatus for effecting sterilization with ultraviolet light.

## DETAILED DESCRIPTION OF THE PREFERRED EMBODIMENT

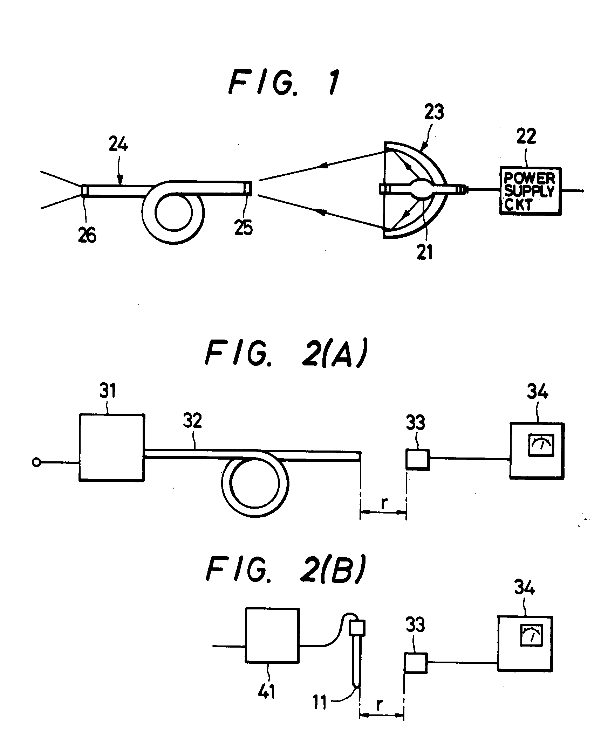

An embodiment of the present invention is hereinafter described with reference to Figs. 1 to 3, in which like reference numerals represent like parts throughout.

As shown in Fig. 1, a mercury-vapor xenon arc lamp 21 is connected to a power supply circuit 22 which supplies power for light emission. The lamp 21 is surrounded by a reflector mirror 23 having a generally elliptical inner surface. A light guide 24 is positioned ahead of the lamp 21. One end surface of the light guide 24 which serves as a light receiving surface 25 is positioned in a face-to-face relationship with the reflector mirror 23, while the other end surface of the light guide 25 which serves as a light emerging surface 26 is directed toward an object to be sterilized (not shown).

Ultraviolet light eminating from the mercury-vapor xenon arc lamp 21 is reflected by the mirror 23 and concentrated at the receiving end surface 25 of the light guide 24. Ultraviolet radiation having a desired wavelength in a range of from 200 to 300 nm (for example, 254 nm) may be selectively reflected from the mirror 23 if the reflecting surface of the mirror 23 has a coating of a multilayered dielectric.

The ultraviolet light incident on the receiving end surface 25 of the light guide 24 propagates through it and emerges from the exit end surface 26. Since the light guide 24 has optical fibers which may be bundled together, the ultraviolet light emerging from the light guide can be directed to a desired position. The light guide 24 is also easy to handle since it can be designed to have a small diameter. It is also, however, rugged in structure.

Various modifications can be made to the apparatus shown in Figure 1 for instance, in addition to a mercury-vapor xenon arc lamp, many other ultraviolet light sources can be used such as a high pressure mercury-vapor lamp, an ultra high-pressure mercury-vapor lamp, and a microwave discharge lamp. High-pressure mercury-vapor lamps and mercury-vapor xenon arc lamps typically emit radiations having wavelengths between 180 and 2,000 nm but, in terms of radiant intensity ($\mu W/cm^2$), they produce strong spectrum lines at such wavelengths as 365, 405, 436, 546 and 577 nm. Therefore, these lamps are generally employed as light sources of near ultraviolet radiations. However, they also produce spectrum lines of low relative intensity in the far ultraviolet region centered on a wavelength of 254 nm. In addition, the absolute value of radiant intensity at 254 nm is sometimes greater than what is produced by ordinary low-pressure mercury-vapor lamps. Therefore, ultraviolet radiation having a wavelength of 254 nm can be effectively used for the purpose of the present invention by selectively reflecting said radiation from the mirror 23.

A low-pressure mercury-vapor lamp can also be used as an ultraviolet lamp, but this lamp has the disadvantage that light issuing therefrom is not easy to concentrate in the light guide with high efficiency because it has a large surface area and produces a low radiant intensity, however it does produce high radiant energy.

As for the reflector mirror 23, one which has an ellipsoidal shape may be used if the ultraviolet lamp is close to a point source in nature such as a high-pressure mercury-vapor lamp or a mercury-vapor xenon arc lamp. If a microwave discharge lamp in a rod shape is used as an ultraviolet lamp, it may be combined with a columnar mirror having a elliptical cross section and a substantially linear longitudinal section. In this case, the trough-shaped mirror is positioned so that its axis is parallel to that of the rod-shaped lamp.

In order to achieve selective reflection of ultraviolet radiation having a predetermined wavelength, the reflector mirror 23 may be coated with several tens of dielectric layers each having a thickness of a quarter of the wavelength. Suitable materials for this dielectric coating include $Al_2O_3/NaF$, $Sc_2O_3/MgF_2$, $ThF_4/Na_2AlF_6$, $HfO_2/SiO_2$, and $PbF_2/Na_3AlF_6$. If desired, the multilayered dielectric coating may be replaced by an aluminum coating. The ultraviolet radiation is not limited to one having a wavelength of in a range of 200 to 300 nm (particularly 254 nm) but may be changed to various wavelengths depending upon the emission spectrum of the ultraviolet lamp used or the absorption spectrum of the object to be sterilized.

The optical fibers in the light guide may have a pure quartz core. Ordinary optical fibers having a core containing germanium oxide suffer from great loss in transmission of wavelengths of 400 nm and below and hence are not generally suitable for use in the embodiment under discussion. However, ultraviolet optical fibers produced by Sumitomo Electric Industry Ltd. (MS and BS series) can be used for commercial applications of the present invention since a length of 1 m of such fibers is capable of transmitting at least 85% of an ultraviolet radiation at a wavelength of 254 nm.

Light guide 24 may employ a bundle of 475 optical fibers having a core diameter of 180$\mu$m. Alternatively, a single optical fiber having a core diameter of 0.8 mm may be used to concentrate ultraviolet light on a small area. In the embodiment under discussion, the dimensions of the area to be irradiated with ultraviolet light are determined by those of the light guide and may be variously adjusted as appropriate for the object to be sterilized.

Examples

In order to verify the effectiveness of the embodiment under discussion, the present inventors conducted the following three experiments.

The first experiment was conducted in order to show the effectiveness of selective reflection by reflector mirrors. According to the present invention, a reflector mirror having a coating of a multilayered dielectric coating for selective reflection of 254 nm was provided. Light from this mirror was concentrated at the receiving end surface of a light guide which was a bundle, one meter long, of 473 optical fibers having a pure quartz core. The intensity of light having a wavelength of 254 nm was measured at a point 10 mm away from the exist end surface of the optical guide. The same measurement was made on an apparatus employing an ordinary reflector mirror. In both measurements, a 100-W mercury-vapor xenon arc lamp was used as the ultraviolet lamp.

The apparatus using the ordinary reflector mirror produced emerging ultraviolet light having an intensity of 7.5 mW/cm$^2$, whereas the apparatus employing the reflector mirror for selective reflection of 254 nm produced emerging ultraviolet light of 700 mW/cm$^2$. It is therefore clear that the selective reflecting mirror achieved nearly 100 times intensity of ultraviolet light as compared with the ordinary reflector mirror.

The second experiment was conducted in order to show the effects of transmission loss of light guides. A reflector mirror for selective reflection of 254 nm was installed on a 100-W mercury-vapor xenon arc lamp and light from this lamp was concentrated at the receiving end surface of two types of light guides. The intensity of light having a wavelength of 254 nm was measured at a point 10 mm away from the exit end surface of each light guide. The first type of light guide was a bundle, one meter long, of 473 optical fibers having a pure quartz core, and the other type of light guide was a bundle, one meter long, of 473 communications optical fibers having a germanium oxide containing core.

The apparatus using optical fibers having a germanium oxide containing core produced emerging ultraviolet light having an intensity of 0.1 mW/cm$^2$ whereas the apparatus using optical fibers having a pure quartz core produced an intensity of 700 mW/cm$^2$. It is therefore clear that the optical fibers having a pure quartz core achieved 7,000 times the intensity of ultraviolet light as compared with the fibers having a germanium oxide containing core.

The third experiment was conducted in order to compare the results of the irradiation of ultraviolet light from the conventional pen-shaped low-pressure mercury-vapor lamp with those of irradiation of ultraviolet light from the apparatus of the present invention. The two types of irradiation systems are shown schematically in FIGURE 2. In the apparatus of the present invention, the combination of a 100-W mercury-vapor xenon arc lamp and a reflective mirror for selective reflection of 254 nm was used as a light source 31 in FIGURE 2(A). The light guide 32 was in the form of a bundle of optical fibers having a pure quartz core. A sensor 33 for detection of ultraviolet radiation of 254 nm was positioned at a distance of r (mm) away from the light guide 32 and the output of the light guide was measured with a circuit 34.

The apparatus employing the conventional technique is shown schematically in FIGURE 2(B). A pen-shaped low-pressure mercury-vapor lamp 11 was excited with a lighting circuit 41 to generate ultraviolet radiation and its output was measured with a circuit 34 using a sensor 33 for detecting ultraviolet radiation at 254 nm that was positioned at a distance of r (mm) away from the lamp 11.

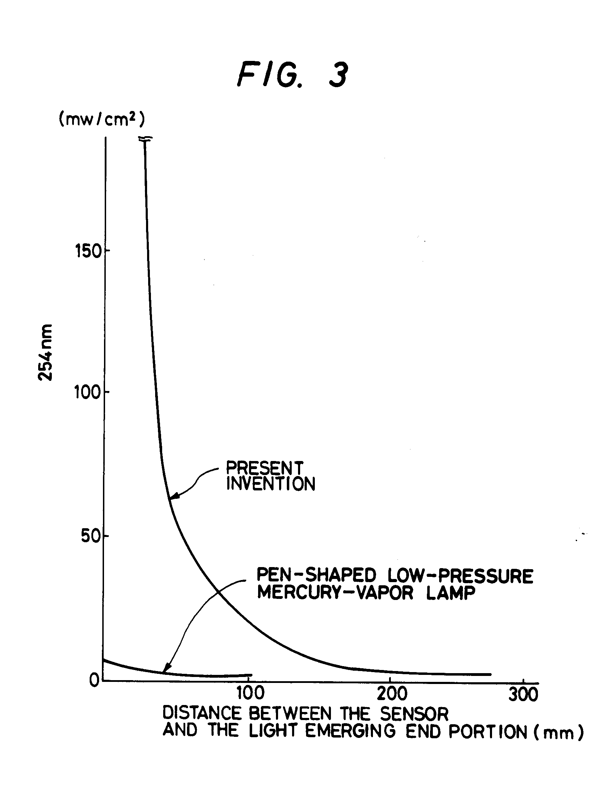

The results of the two measurements are shown in FIGURE 3, from which one can see that a very high intensity of ultraviolet radiation at 254 nm can be attained by the present invention.

Although a preferred embodiment is specifically illustrated and described herein, it will be appreciated that many modifications and variations of the present invention are possible in light of the above teachings and within the purvue of the appended claims, without departing from the spirit and intended scope of the invention.

**Claims**

1. An apparatus for sterilization with ultraviolet light which comprises: an ultraviolet lamp (21); **characterised by** further comprising a light guide (24) having optical fibers, said light guide having a receiving end surface (25) and an exit end surface (26); and a reflector mirror (23) which reflects a predetermined wavelength of ultraviolet radiation from the ultraviolet lamp and concentrates the reflected radiation at the receiving end surface of said light guide, the light emerging from the exit end surface of said light guide being directed on an object to be sterilized.

2. A sterilization apparatus as in claim 1 wherein said ultraviolet lamp is a high-pressure mercury-vapor lamp.

3. A sterilization apparatus according to claim 1 wherein said ultraviolet lamp is a mercury-vapor xenon arc lamp.

4. A sterilization apparatus according to claim 1 wherein said ultraviolet lamp is an ultrahigh-pressure mercury-vapor lamp.

5. A sterilization apparatus according to claim 1 wherein said ultraviolet lamp is a microwave discharge lamp.

6. A sterilization apparatus according to claim 1 wherein said reflector mirror has a coating of a multilayered dielectric film.

7. A sterilization apparatus according to claim 1 wherein said reflector mirror has an aluminum coating.

8. A sterilization apparatus according to claim 1 wherein said reflector mirror is capable of selectively reflecting an ultraviolet radiation at a wavelength of in a range of from 200 to 300 nm.

9. A sterilization apparatus according to claim 8 wherein said reflector mirror is capable of selectively reflecting an ultraviolet radiation at a wavelength of 254 nm.

10. A sterilization apparatus according to claim 1 wherein the optical fibers in said light guide have a pure quartz core.

**Revendications**

1. Appareil de stérilisation par lumière ultraviolette comprenant : une lampe à rayons ultraviolets (21) ; caractérisé en ce qu'il comprend en outre un guide de lumière (24) comportant des fibres optiques, ledit guide de lumière comprenant une surface d'extrémité de réception (25) et une surface d'extrémité de sortie (26) ; et un miroir réflecteur (23) qui réfléchit une longueur d'onde prédéterminée du rayonnement ultraviolet qui provient de la lampe à rayons ultraviolets et qui concentre le rayonnement réfléchi au niveau de la surface d'extrémité de réception dudit guide de lumière, la lumière qui émerge de la surface d'extrémité de sortie dudit guide de lumière étant dirigée sur un objet qui doit être stérilisé.

2. Appareil de stérilisation selon la revendication 1, dans lequel ladite lampe à rayons ultraviolets est une lampe à vapeur de mercure haute pression.

3. Appareil de stérilisation selon la revendication 1, dans lequel ladite lampe à rayons ultraviolets est une lampe à arc à xénon et vapeur de mercure.

4. Appareil de stérilisation selon la revendication 1, dans lequel ladite lampe à rayons ultraviolets est une lampe à vapeur de mercure à ultra-haute pression.

5. Appareil de stérilisation selon la revendication 1, dans lequel ladite lampe à rayons ultraviolets est une lampe à décharge micro-ondes.

6. Appareil de stérilisation selon la revendication 1, dans lequel ledit miroir réflecteur comporte un revêtement constitué par un film diélectrique multi-couches.

7. Appareil de stérilisation selon la revendication 1, dans lequel ledit miroir réflecteur comporte un revêtement en aluminium.

8. Appareil de stérilisation selon la revendication 1, dans lequel ledit miroir réflecteur peut réfléchir de manière sélective un rayonnement ultraviolet à une longueur d'onde qui se situe dans une plage qui va de 200 à 300 nm.

9. Appareil de stérilisation selon la revendication 8, dans lequel ledit miroir réflecteur peut réfléchir de manière sélective un rayonnement ultraviolet à une longueur d'onde de 254 nm.

10. Appareil de stérilisation selon la revendication 1, dans lequel les fibres optiques contenues dans ledit guide de lumière comprennent une âme en quartz pur.

**Patentansprüche**

1. Eine Vorrichtung zum Sterilisieren mit ultraviolettem Licht, welche eine Ultraviolettlampe (21) umfaßt; **dadurch gekennzeichnet,** daß die Vorrichtung ferner eine optische Fasern aufweisende Lichtführung (24), wobei die Lichtführung eine Empfangsendoberfläche (25) und eine Ausgangsendoberfläche (26) aufweist; und einen Reflektorspiegel (23) umfaßt, welcher eine vorbestimmte Wellenlänge der ultravioletten Strahlung aus der Ultraviolettlampe reflektiert und die reflektierte Strahlung auf der Empfangsendoberfläche der Lichtführung konzentriert, wobei das aus der Ausgangsendoberfläche der Lichtführung austretende Licht auf ein zu sterilisierendes Objekt gerichtet wird.

2. Eine Sterilisationsvorrichtung nach Anspruch 1, wobei die Ultraviolettlampe eine Hochdruckquecksilberdampflampe ist.

3. Eine Sterilisationsvorrichtung nach Anspruch 1, wobei die Ultraviolettlampe eine Quecksilberdampfxenonlichtbogenlampe ist.

4. Eine Sterilisationsvorrichtung nach Anspruch 1, wobei die Ultraviolettlampe eine Ultrahochdruckquecksilberdampflampe ist.

5. Eine Sterilisationsvorrichtung nach Anspruch 1, wobei die Ultraviolettlampe eine Mikrowellenentladungslampe ist.

6. Eine Sterilisationsvorrichtung nach Anspruch 1, wobei der Reflektorspiegel einen Überzug aus einem dielektrischen, eine Vielzahl von Schichten umfassenden Film aufweist.

7. Eine Sterilisationsvorrichtung nach Anspruch 1, wobei der Reflektorspiegel eine Aluminiumbeschichtung aufweist.

8. Eine Sterilisationsvorrichtung nach Anspruch 1, wobei der Reflektorspiegel in der Lage ist, selektiv eine Ultraviolettstrahlung bei einer Wellenlänge im Bereich von 200 bis 300 nm zu reflektieren.

9. Eine Sterilisationsvorrichtung nach Anspruch 1, wobei der Reflektorspiegel in der Lage ist, selektiv eine Ultraviolettstrahlung bei einer Wellenlänge von 254 nm zu reflektieren.

10. Eine Sterilisationsvorrichtung nach Anspruch 1, wobei die optischen Fasern in der Lichtführung einen Kern aus reinem Quarz aufweisen.

# F I G. 1

# F I G. 2(A)

# F I G. 2(B)

## FIG. 3

(mw/cm²)

254nm

150

100

50

0

PRESENT
INVENTION

PEN-SHAPED LOW-PRESSURE
MERCURY-VAPOR LAMP

100        200        300

DISTANCE BETWEEN THE SENSOR
AND THE LIGHT EMERGING END PORTION (mm)

# FIG. 4
## PRIOR ART

# FIG. 5
## PRIOR ART